Europäisches Patentamt

European Patent Office

Office européen des brevets

(19)

(11) Publication number: **0 121 366**
**B1**

# EUROPEAN PATENT SPECIFICATION

(12)

(45) Date of publication of patent specification: 04.11.87

(51) Int. Cl.⁴: **C 07 D 307/66**

(21) Application number: **84301761.7**

(22) Date of filing: **15.03.84**

(54) **Process for the preparation of N-formylaspartic anhydride.**

(30) Priority: **25.03.83 JP 50245/83**

(43) Date of publication of application:
**10.10.84 Bulletin 84/41**

(45) Publication of the grant of the patent:
**04.11.87 Bulletin 87/45**

(84) Designated Contracting States:
**BE CH DE FR GB IT LI NL**

(56) References cited:
**FR-A-2 078 640**

**CHEMICAL ABSTRACTS, vol. 86, no. 1, 3rd
January 1977, page 510, no. 5850n, Columbus,
Ohio, US**

(73) Proprietor: **AJINOMOTO CO., INC.
5-8, Kyobashi 1-chome, Chuo-ku
Tokyo 104 (JP)**

(72) Inventor: **Takemoto, Tadashi
No. 1155-2, Nakamaruko Nakahara-ku
Kawasake-Shi Kanahawa-ken (JP)**
Inventor: **Hijiya, Toyoto
Toyobiru B-304 No.2-210-1, Negishi-cho
Yokosuka-shi Kanagawa-ken (JP)**
Inventor: **Yamatani, Tetsuo
No. 5380, Hinaga
Yokkaichi-shi Mie-ken (JP)**

(74) Representative: **Bond, Bentley George et al
Haseltine Lake & Co. 28 Southampton Buildings
Chancery Lane
London WC2A 1AT (GB)**

Courier Press, Leamington Spa, England.

# 0 121 366

**Description**

This invention relates to a process for the preparation of N-formylaspartic anhydride from aspartic acid. The aspartic acid may be in an optically active form or in a racemic form.

A process for the preparation of N-formylaspartic anhydride by reacting formic acid and acetic anhydride with aspartic acid is known from Published Unexamined Japanese Patent Application SHO 46(1971)-1370. In this reaction, there is generally used excess formic acid. When N-formylaspartic anhydride is manufactured on a commercial basis, therefore, the excess formic acid must be recovered and put to reuse. Incidentally, the aforementioned reaction, by the time of its completion, produces acetic acid in an amount of 2 moles per mole of acetic anhydride consumed. The recovery of formic acid, therefore, inevitably entails separation of formic acid from an acetic acid-formic acid mixed system. As means for this separation, there is known a method wherein the separation is effected by direct distillation of the mixed system, and a method wherein the separation is effected by first adding an organic solvent such as toluene to the mixed system and then subjecting the resultant three-component system to distillation. Neither of these methods permits easy separation of pure formic acid. To be more specific, they inevitably suffer from the presence in the separated formic acid of extraneous substances, namely acetic acid in the former method and acetic acid and the organic solvent such as toluene in the latter method.

In the known process for the preparation of N-formylaspartic anhydride, pure formic acid is used. Thus, the known method does not involve the use of formic acid containing acetic acid (or acetic acid and an organic solvent such as toluene). The inventors carried out the reaction for the production of N-formylaspartic anhydride, using formic acid containing acetic acid (or acetic acid and an organic solvent such as toluene), and have found that the product is obtained in significantly lower yields than when pure formic acid is used.

N-formyl-L-aspartic anhydride is used as the raw material for the manufacture of α-L-aspartyl-L-phenylalanine methyl ester, which is used as a sweetening agent. When α-L-aspartyl-L-phenylalanine methyl ester is manufactured on a commercial basis, the portion of N-formyl-L-aspartic anhydride which has not been utilized as the aspartic acid component of the product must be recovered in the form of L-aspartic acid and put to reuse. The inventors have carried out the process for the preparation of N-formylaspartic anhydride, using the L-aspartic acid thus recovered, and have found that the product of the reaction is obtained in significantly lower yields than when commercially available L-aspartic acid is used.

The inventors, therefore, conducted a diligent study with a view to developing a commercially advantageous process for the preparation of N-formylaspartic anhydride without entailing the drawbacks mentioned above.

Thus, according to the present invention, there is provided a process for the preparation of N-formylaspartic anhydride, which comprises reacting formic acid and acetic anhydride with aspartic acid, characterized in that the reaction is carried out in the presence of an oxide, a hydroxide or a salt of a metal.

The process of this invention, when used for the preparation of N-formlyaspartic anhydride from formic acid containing acetic acid (or acetic acid and an organic solvent such as toluene), or for the preparation of N-formylaspartic anhydride from L-aspartic acid recovered from a process for the production of α-L-aspartyl-L-phenylalanine methyl ester, or for the preparation of N-formylaspartic anhydride from both of the raw materials mentioned above, enables the desired compound to be produced in high yields, by virtue of the additional use in the reaction system of an oxide or a hydroxide of various metals or a salt of such a metal with various acids.

The amount of acetic anhydride used in this invention is preferably at least 2 moles per mole of aspartic acid. If an amount of less than 2 moles is used, the formylation and the anhydridization might not proceed thoroughly.

The amount of acetic anhydride has no specific upper limit. If acetic anhydride is used in excess, there results a proportional increase in the amount of excess acetic anhydride to be recovered. Such use of excess acetic anhydride is not commercially advantageous. Thus, the amount is preferably in the range of 2 to 2.5 moles.

The amount of formic acid used, calculated as pure formic acid, is preferably from 2 to 3 moles per mole of aspartic acid. If formic acid is used in an amount exceeding 3 moles, there results a proportional increase in the amount of excess formic acid to be recovered. Such use of excess formic acid is not commercially advantageous.

The organic solvent which may be contained in the formic acid may be of any kind provided that it is inert to the reactants and to the product of the reaction, and provided that it is capable of effectively functioning as an entrainer in the separation of formic acid from the formic acid-acetic acid mixed system by distillation. Typical examples are hydrocarbons such as toluene, xylene and hexane, halogenated hydrocarbons such as chloroform and ethylene dichloride, esters such as ethyl acetate and methyl propionate, and ketones such as acetone and methylethyl ketone.

The reaction temperature, with a view to curbing possible racemization of the product of the reaction to the fullest extent, is generally in the range of not more than 100°C to not less than −10°C, preferably in the range of not more than 80°C to not less than 0°C.

Examples of the metal compound to be added to the reaction system are oxides and hydroxides of

2

various metals such as alkali metals (e.g. lithium, sodium and potassium), alkaline earth metals (e.g. magnesium and calcium), copper-family elements (e.g. copper), zinc-family elements (e.g. zinc), boron-family elements (e.g. aluminium), and iron-family elements (e.g. iron); and salts of such metals with various acids, including carbonates, acetates and other carboxylates, hydrochlorides, hydrobromides, nitrates, phosphates, and sulphates. Although the amount of the metal compound used is not specifically limited, it is desirably such as not to adversely affect any step following the reaction process. The maximum amount of the metal compound tolerated depends to some extent on the particular compound used. In the case of magnesium acetate, for instance, the amount is preferably 0.0087 mole per mole of L-aspartic acid. Thus, the metal compound manifests its effect even in such a small amount.

The preferred amounts of such compounds which are additionally incorporated in the reaction system, on a commercial scale, can be easily found by any person of ordinary skill in the art by conducting a preliminary experiment of the reaction involved. The addition of such compounds to the reaction system is generally made prior to the start of the anhydridization. Optionally, it may be effected during the course of the reaction.

As described above, the process of this invention enables N-formylaspartic anhydride, e.g. N-formyl-L-aspartic anhydride, to be produced in high yields even when the formic acid contains acetic acid (or acetic acid and an organic solvent such as toluene) and even when aspartic acid recovered from a process for the production of α-L-aspartyl-L-phenylalanine methyl ester is used.

The invention will now be illustrated by the following Examples.

Example 1

A solution prepared by adding 21 ml (0.21 mole) of acetic anhydride to 9.8 ml (0.35 mole) of formic acid, 3.3 ml of acetic acid and 3.3 ml of toluene was stirred with 13.3 g (0.1 mole) of L-aspartic acid, and 0.187 g ($8.7 \times 10^{-4}$ mole) of magnesium acetate tetrahydrate was added thereto. The solution was kept at 45°C for 3.5 hours, to effect reaction.

The resultant slurry was stirred with 58 ml of toluene added thereto as kept cooled with ice for one hour. The mixture consequently formed was subjected to suction filtration. There were obtained 13.4 g of N-formyl-L-aspartic anhydride in a crystalline form. The yield was 94.0%. The melting point and the infrared absorption spectrum of this compound agreed with those of a standard sample of N-formyl-L-aspartic anhydride.

Separately, the slurry obtained by repeating the above reaction was concentrated under a vacuum to expel the solvent. The residue of the distillation was dissolved in 100 ml of methanol, which was added thereto. The resultant solution was quantitatively separated by high-speed liquid chromatography into N-formyl-L-aspartic acid-α-methyl ester and N-formyl-L-aspartic acid-β-methyl ester. Since the N-formyl-L-aspartic anhydride had reacted with methanol to produce the α and β methyl esters, the yield of N-formyl-L-aspartic anhydride could be calculated, and was found to be 98.0%.

Example 2

In a mixture consisting of 9.8 ml (0.25 mole) of formic acid and 21 ml (0.21 mole) of acetic anhydride, 14.0 g (0.1 mole) of L-aspartic acid (95% pure, recovered from a process for the production of α-L-aspartyl-L-phenylalanine methyl ester) and 0.187 g ($8.7 \times 10^{-4}$ mole) of magnesium acetate tetrahydrate were stirred. The mixture was kept at 45°C for 3.5 hours to effect reaction.

The resultant slurry was concentrated under a vacuum to expel the solvent. The residue of the distillation was dissolved in 100 ml of methanol, which was added thereto, and the solution consequently obtained was quantitatively analyzed by the procedure of Example 1.

The yield of N-formyl-L-aspartic anhydride was 97.8%.

Comparative Experiment 1

When the reaction of Example 1 was carried out without the addition of magnesium acetate, the yield of N-formyl-L-aspartic anhydride was only 85.0%.

Comparative Experiment 2

When the reaction of Example 2 was carried out without the addition of magnesium acetate, the yield of N-formyl-L-aspartic anhydride was only 83.8%.

Example 3

When the procedure of Example 1 was repeated, using L-aspartic acid having a purity of 95% and recovered from a process for the production of α-L-aspartyl-L-phenylalanine methyl ester, the yield of N-formyl-L-aspartic anhydride was 97.5%.

Example 4

When the procedure of Example 1 was repeated, using hexane in place of toluene, the yield of N-formyl-L-aspartic anhydride was 97.8%.

Example 5

When the procedure of Example 1 was repeated, using ethyl acetate in place of toluene, the yield of N-formyl-L-aspartic anhydride was 98.2%.

Examples 6—9

Reactions were carried out in the presence of the various compounds indicated in Table 1. The conditions, except those indicated in Table 1, and the procedure were similar to those of Example 1.

TABLE 1

| Example | Compound added | Amount added (g) | Yield of reaction (%) |
|---------|----------------|------------------|----------------------|
| 6 | $K \cdot OCOCH_3$ | 0.080 | 95.6 |
| 7 | $MgCl_2 \cdot 6H_2O$ | 0.177 | 98.2 |
| 8 | $Ca(OCOCH_3)_2 \cdot H_2O$ | 0.153 | 98.0 |
| 9 | $Zn(OCOCH_3)_2 \cdot 2H_2O$ | 0.191 | 98.6 |

Examples 10—13

Reactions were carried out in the presence of the various compounds indicated in Table 2. The conditions, other than those indicated in Table 2, and the procedure were similar to those of Example 2.

TABLE 2

| Example | Compound added | Amount added (g) | Yield of reaction (%) |
|---------|----------------|------------------|----------------------|
| 10 | $K \cdot OCOCH_3$ | 0.080 | 96.8 |
| 11 | $MgCl_2 \cdot 6H_2O$ | 0.177 | 97.5 |
| 12 | $Ca(OCOCH_3)_2 \cdot H_2O$ | 0.153 | 97.8 |
| 13 | $Zn(OCOCH_3)_2 \cdot 2H_2O$ | 0.191 | 97.3 |

**Claims**

1. A process for the preparation of N-formyl-aspartic anhydride, which comprises reacting formic acid and acetic anhydride with aspartic acid, characterized in that the reaction is carried out in the presence of an oxide, a hydroxide or a salt of a metal.

2. A process according to claim 1, wherein the metal is an alkali metal, an alkaline earth metal, a copper-family metal, a zinc-family metal, a boron-family metal, or an iron-family metal.

3. A process according to claim 1 or 2, wherein the salt is a carbonate, a carboxylate, a hydrochloride, a hydrobromide, a nitrate, a phosphate, or a sulphate.

4. A process according to any of claims 1 to 3, wherein the acetic anhydride is used in an amount of at least 2 moles per mol of aspartic acid.

5. A process according to claim 4, wherein the acetic anhydride is used in an amount of from 2 to 2.5 moles per mole of aspartic acid.

6. A process according to any of claims 1 to 5, wherein the formic acid is used in an amount of from 2 to 3 moles per mol of aspartic acid.

7. A process according to any of claims 1 to 6, wherein the formic acid contains acetic acid, or acetic acid and inert organic solvent.

8. A process according to any of claims 1 to 7, wherein the aspartic acid is L-aspartic acid which has been recovered from a process for the preparation of α-L-aspartyl-L-phenylalanine methyl ester.

9. A process according to any of claims 1 to 8, wherein the reaction is carried out at a temperature of from −10° to 100°C.

**Patentansprüche**

1. Verfahren zur Herstellung von N-Formylasparaginsäureanhydrid, bei dem Ameisensäure und Essigsäureanhydrid mit Asparaginsäure umgesetzt werden, dadurch gekennzeichnet, daß die Reaktion in Gegenwart eines Oxids, eines Hydroxids oder eines Salzes eines Metalls durchgeführt wird.

2. Verfahren nach Anspruch 1, bei dem das Metall ein Alkalimetall, ein Erdalkalimetall, ein Metall der Kupfergruppe, ein Metall der Zinkgruppe, ein Metall der Borgruppe oder ein Metall der Eisengruppe ist.

3. Verfahren nach Anspruch 1 oder 2, bei dem das Salz ein Carbonat, ein Carboxylat, ein Hydrochlorid, ein Hydrobromid, ein Nitrat, ein Phosphat oder ein Sulfat ist.

4. Verfahren nach einem der Ansprüche 1 bis 3, bei dem das Essigsäureanhydrid in einer Menge von mindestens 2 Mol pro Mol Asparaginsäure eingesetzt wird.

5. Verfahren nach Anspruch 4, bei dem das Essigsäureanhydrid in einer Menge von 2 bis 2,5 Mol pro Mol Asparaginsäure eingesetzt wird.

6. Verfahren nach einem der Ansprüche 1 bis 5, bei dem die Ameisensäure in einer Menge von 2 bis 3 Mol pro Mol Asparaginsäure eingesetzt wird.

7. Verfahren nach einem der Ansprüche 1 bis 6, bei dem die Ameisensäure Essigsäure oder Essigsäure und ein inertes organisches Lösungsmittel enthält.

8. Verfahren nach einem der Ansprüche 1 bis 7, bei dem die Asparaginsäure L-Asparaginsäure ist, die aus einem Verfahren zur Herstellung von α-L-Aspartyl-L-phenylalanin-methylester zurückgewonnen wurde.

9. Verfahren nach einem der Ansprüche 1 bis 8, bei dem die Reaktion bei einer Temperatur von −10° bis 100°C durchgeführt wird.

## Revendications

1. Un procédé pour la préparation de l'anhydride N-formylaspartique qui consiste à faire réagir l'acide formique et l'anhydride acétique avec l'acide aspartique, caractérisé en ce que la réaction est mise en oeuvre en présence d'un oxyde, d'un hydroxyde ou d'un sel d'un métal.

2. Un procédé selon la revendication 1, dans lequel le métal est un métal alcalin, un métal alcalino-terreux, un métal de la famille du cuivre, un métal de la famille du zinc, un métal de la famille du bore ou un métal de la famille du fer.

3. Un procédé selon la revendication 1 ou 2, dans lequel le sel est un carbonate, un carboxylate, un chlorhydrate, un bromhydrate, un nitrate, un phosphate ou un sulfate.

4. Un procédé selon l'une quelconque des revendications 1 à 3, dans lequel l'anhydride acétique est utilisé en quantité d'au moins 2 mol par mol d'acide aspartique.

5. Un procédé selon la revendication 4, dans lequel l'anhydride acétique est utilisé en quantité de 2 à 2,5 mol par mol d'acide aspartique.

6. Un procédé selon l'une quelconque des revendications 1 à 5, dans lequel l'acide formique est utilisé en quantité de 2 à 3 mol par mol d'acide aspartique.

7. Un procédé selon l'une quelconque des revendications 1 à 6, dans lequel l'acide formique contient de l'acide acétique, ou de l'acide acétique et un solvant organique inerte.

8. Un procédé selon l'une quelconque des revendications 1 à 7, dans lequel l'acide aspartique est l'acide L-aspartique qui a été récupéré d'un procédé pour la préparation de l'ester méthylique d'α-L-aspartyl-L-phénylalanine.

9. Un procédé selon l'une quelconque des revendications 1 à 8, dans lequel la réaction est mise en oeuvre à une température de −10 à 100°C.